# EUROPÄISCHE PATENTSCHRIFT

(11) **EP 2 485 706 B1**
(45) Veröffentlichungstag und Bekanntmachung des Hinweises auf die Patenterteilung: **19.10.2016**
(21) Anmeldenummer: 10732308.1
(22) Anmeldetag: 13.07.2010
(51) Int. Cl.: A61K 8/34, A61Q 19/00, A61K 8/06

(54) **VERWENDUNG VON 4-N-BUTYLRESORCIN ZUR VERHINDERUNG ODER MINDERUNG DER DIFFUSION EINES ODER MEHRERER BESTANDTEILE KOSMETISCHER ZUBEREITUNGEN, INSBESONDERE EMULSIONEN IN DAS DIE ZUBEREITUNG UMGEBENDE BEHÄLTERMATERIAL**
USE OF 4-N-BUTYLRESORCINOL FOR PREVENTING OR REDUCING THE DIFFUSION OF ONE OR MORE COMPONENTS OF COSMETIC PREPARATIONS, ESPECIALLY EMULSIONS, INTO THE CONTAINER MATERIAL, ESPECIALLY TUBES, SURROUNDING THE PREPARATION
UTILISATION DE 4-N-BUTYLRÉSORCINE POUR EMPÊCHER OU DIMINUER LA DIFFUSION D'UN OU DE PLUSIEURS INGRÉDIENTS DE PRÉPARATIONS COSMÉTIQUES, NOTAMMENT D'ÉMULSIONS DANS LE MATÉRIAU DES CONTENANTS ENTOURANT LA PRÉPARATION, NOTAMMENT DES TUBES

(30) Priorität: 09.10.2009 DE 102009048975
(43) Veröffentlichungstag der Anmeldung: 15.08.2012
(73) Patentinhaber: Beiersdorf AG, 20253 Hamburg (DE)
(72) Erfinder: SCHERNER, Cathrin, 22844 Norderstedt (DE); KOLBE, Ludger, 21255 Dohren (DE); MANN, Tobias, 22175 Hamburg (DE); GERWAT, Wolfram, 22309 Hamburg (DE)
(86) Internationale Anmeldenummer: PCT/EP2010/004248
(87) Internationale Veröffentlichungsnummer: WO 2011/042074

(56) Entgegenhaltungen:
- WO-A1-01/56536
- WO-A1-03/080009
- WO-A1-03/080011

## Beschreibung

Die vorliegende Erfindung betrifft die Verwendung von 4-n-Butylresorcin zur Verhinderung oder Verminderung der Diffusion eines oder mehrerer Bestandteile kosmetischer Emulsionen in das die Zubereitung umgebenden Behältermaterial, insbesondere Tuben.

Übliche kosmetische Darreichungsformen sind Emulsionen. Darunter versteht man im allgemeinen ein heterogenes System aus zwei miteinander nicht oder nur begrenzt mischbaren Flüssigkeiten, die üblicherweise als Phasen bezeichnet werden. Die eine liegt dabei in Form von Tröpfchen vor (dispere oder innere Phase), während die andere Flüssigkeit eine kontinuierliche (kohärente oder innere Phase ) bildet. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Neuere Erkenntnisse führten in letzter Zeit zu einem besseren Verständnis praxisrelevanter kosmetischer Emulsionen. Dabei geht man davon aus, daß die im Überschuß eingesetzten Emulgatorgemische lamellare flüssigkristalline Phasen bzw. kristalline Gelphasen ausbilden. In der Gelnetzwerktheorie werden Stabilität und physikochemische Eigenschaften solcher Emulsionen auf die Ausbildung von viskoelastischen Gelnetzwerden zurückgeführt.

Kosmetische Zubereitungen mit 4-n-Butylresorcin sind bekannt, beispielsweise aus der EP 1 490017.

4-n-Butylresorcin wird auch Rucinol oder Lucinol genannt. Es hemmt die Melaninproduktion, indem es das für die Melaninsynthese (Melanogenese) erforderliche Enzym, die Tyrosinase, hemmt. Es wird zunächst die Melaninproduktion gehemmt, dann die Produktion des schwarzen Melanins, das für die intensive Färbung der Pigmentflecken verantwortlich ist, blockiert.

Enthalten Tuben kosmetische Grundlagen, diffundiert ein gewisser Anteil der kosmetischen Grundlagen in die Tuben. Hierdurch werden die Materialeigenschaften der Tuben teils erheblich verändert.

Überraschenderweise konnte aufgezeigt werden, daß kosmetische Grundlagen, die 4-n-Butylresorcin enthalten, zu einem geringeren Maße in das Behältermaterial diffundieren, so daß derartige Nachteile vermieden werden können.

Gegenstand der Erfindung ist also die Verwendung von 4-n-Butylresorcin zur Verhinderung oder Verminderung der Diffusion eines oder mehrerer Bestandteile Emulsionen in das die Zubereitung umgebenden Behältermaterial, insbesondere Tuben.

4-n-Butylresorcin , CAS [18979-61-8], ist durch die chemische Struktur 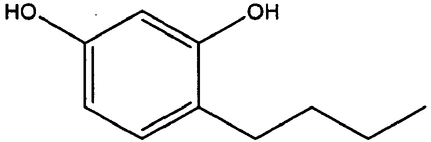 gekennzeichnet.

Bevorzugt enthalten kosmetische oder dermatologische Zubereitungen gemäß der Erfindung 0,001 - 10 Gew.-%, besonders bevorzugt 0,01 - 1 Gew.-%, an 4-n-Butylresorcin, bezogen auf die Gesamtzusammensetzung der Zubereitungen.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die kosmetischen und dermatologischen Zubereitungen können erfindungsgemäß kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an üblichen Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die kosmetischen oder dermatologischen Zubereitung im Sinne der vorliegenden Erfindung, können als Lösungsmittel enthalten:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Kosmetische Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.%, enthalten.

Es ist erfindungsgemäß vorteilhaft, außer den erfindungsgemäßen Kombinationen weitere öllösliche UVA-Filter und/oder UVB-Filter in der Lipidphase und/oder weitere wasserlösliche UVA-Filter und/oder UVB-Filter in der wäßrigen Phase einzusetzen.

Vorteilhaft können die Lichtschutzformulierungen erfindungsgemäß weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Anhand des nachfolgenden Versuches soll die Wirksamkeit der vorliegenden Erfindung demonstriert werden.

Angewendet wurde die TGA-Methode, in der die Gewichtsabnahme der kosmetischen Grundlage (bzw. die Gewichtszunahme des Packmittels) untersucht wurde.

### B214 enthält 0.3% 4-n-Butylresorcin

B215 stellt die gleiche Grundlage, jedoch ohne 4-n-Butylresorcin dar.

### Methodenbeschreibung:

Thermogravimetrische Bestimmung mit einer TGA/ SDTA 851e LF (0,1µg) von Mettler Toledo:

| | |
|---|---|
| Gerät: | Mettler TGA 851 e LF (0,1µg) |
| Messung: | Temperaturrampe mit Blindkurve und Isothermphase |
| Messsystem: | Al-Tiegel 40µl |
| Messtemperatur: | 25°C → 350°C = 60min ISO |
| Heizrate: | 50°C/min |
| Spülgas: | Stickstoff 5.0 |

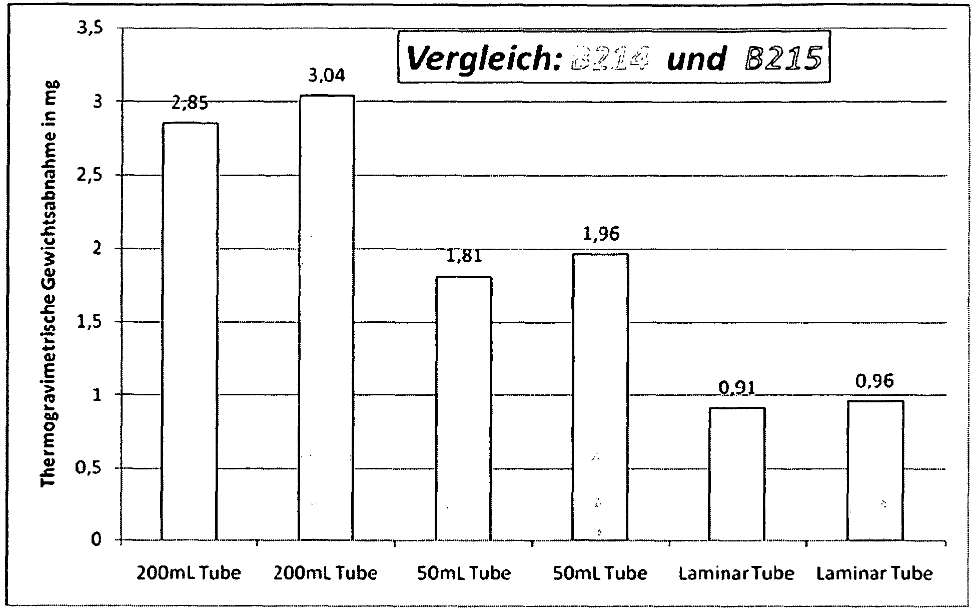

| **Beispielrezeptur B214** | Gew. % |
|---|---|
| Methylparaben | 0.2 |
| Propylparaben | 0.1 |
| Glycerylstearat | 2.6 |
| Caprylsäure/Caprinsäuretriglycerid | 2.5 |
| Cetalstearyl Alkohol | 3 |
| PEG-40 Stearat | 0.8 |
| Phenoxyethanol | 0.4 |
| Dimethicon | 0.4 |
| Cyclomethicon | 2 |
| C12-15 Alkylbenzoat | 2.5 |
| Glycerin | 9 |
| Wasser + NaOH | 0.02 |
| Butylenglycol | 3 |
| Carbomer | 0.1 |
| Natriumpolyacrylat | 0.2 |
| Dicaprylylcarbonat | 2.5 |
| 4-n-Butylresorcin | 0.3 |
| Wasser | ad 100,00 |

| **Beispielrezeptur B215** | Gew. % |
|---|---|
| Methylparaben | 0.2 |
| Propylparaben | 0.1 |
| Glycerylstearat | 2.6 |
| Cäprylsäure/Caprinsäuretriglycerid | 2.5 |
| Cetalstearyl Alcohol | 3 |
| PEG-40 Stearat | 0.8 |
| Phenoxyethanol | 0.4 |
| Dimethicon | 0.4 |
| Cyclomethicon | 2 |
| C12-15 Alkylbenzoat | 2.5 |
| Glycerin | 9 |
| Wasser + NaOH | 0.02 |
| Butylenglycol | 3 |
| Carbomer | 0.1 |
| Natriumpolyacrylat | 0.2 |
| Dicaprylylcarbonat | 2.5 |
| Wasser | ad 100,00 |

| **INCl - Bezeichnung** | **Gew.-%** |
|---|---|
| Glyceryl Stearate Citrat | 2,00 |
| Behenyl Alkohol | 1,00 |
| C12-15 Alkyl Benzoat | 2,50 |
| Caprylic/Capric Triglycerid | 2,50 |
| Cetyl Alcohol | 2,00 |
| Cyclomethicon | 2,00 |
| Dicaprylyl Carbonat | 2,50 |
| Dimethicon | 1,00 |
| Glycerin | 6,00 |
| Methylparaben | 0,20 |
| Phenoxyethanol | 0,40 |
| Propylparaben | 0,10 |
| Carbomer | 0,20 |
| Natrium Polyacrylat | - |
| Xanthan Gum | 0,10 |
| Puderrohstoffe | 0,50 |
| 4-Butyl-Resorcin | 0,50 |
| Natrium Metabisulfit | 0,10 |
| Diethylhexyl Syringylidenemalonat + Caprylic/Capric Triglycerid | 0,10 |
| Titaniumdioxid | - |
| Octocrylene | - |
| Bis-Ethylhexyloxyphenol Methoxyphenyl Triazines | - |
| Natriumhydroxide | q.s. |
| Parfume | q.s. |
| Wasser | ad 100 |

| **INCl - Bezeichnung** | **Gew.-%** |
|---|---|
| Polyglyceryl-3 Methylplucose Distearat | 2,50 |
| Sorbitan Stearat | 3,00 |
| C12-15 Alkyl Benzoat | 2,50 |
| Caprylic/Capric Triglycerid | 2,50 |
| Stearyl Alkohol | 1,50 |
| Cyclomethicon | 1,00 |
| Dicaprylyl Carbonat | 2,50 |
| Dimethicon | 1,00 |
| Glycerin | 7,50 |
| Methylparaben | 0,20 |
| Phenoxyethanol | 0,40 |
| Propylparaben | 0,10 |
| Carbomer | 0,10 |
| Trinatrium EDTA + Wasser | 1,00 |
| Tapioca Stärke | 1,00 |
| Distarch phosphat | 1,00 |
| 4-Butyl-Resorcin | 0,30 |
| Titaniumdioxid | 0,50 |
| Butyl Methoxydibenzoylmethan | 1,00 |
| Octocrylene | 1,00 |
| Natriumhydroxide | q.s. |
| Parfume | q.s. |
| Wasser | ad 100 |

| **INCI - Bezeichnung** | **Gew.-%** |
|---|---|
| Sucrose Polystearat + Hydrogynated Polyisobutene | 1,00 |
| Natrium Stearoyl Glutamat | 0,20 |
| C12-15 Alkyl Benzoat | 1,50 |
| Cetyl Alkohol | 0,50 |
| Cyclomethicon | 10,00 |
| Dimethicon | 3,00 |
| Glycerin | 7, 50 |
| Isopropyl Stearat | 1,00 |
| Paraffinum Liquidum | 3,00 |
| Methylparaben | 0,10 |
| Propylparaben | 0,10 |
| Phenoxyethanol | 0,20 |
| 4-Butyl-Resorcin | 0,50 |
| Bis-Ethylhexyloxyphenol Methoxyphenyl Triazin | 1,00 |
| Butyl Methoxydibenzoylmethan | 2,00 |
| Ammonium Acryloyldimethyltaurate/VP Copolymer | 0,10 |
| Xanthan Gum | 0,10 |
| Acrylates/C10-30 Alkyl Acrylate Crosspolymer | 0,25 |
| Parfum | q.s. |
| Natriumhydroxide | q.s. |
| Wasser | ad 100 |

| | |
|---|---|
| Anmerkung: Sucrosepolystearat + Hydrogeniertes Polyisobuten - eingesetzt wurde die Mischung Emulgade Sucro™ der Firma Cognis. | |

## Patentansprüche

1. Verwendung von 4-n-Butylresorcin zur Verhinderung oder Verminderung der Diffusion eines oder mehrerer Bestandteile kosmetischer Emulsionen in das die Zubereitung umgebenden Behältermaterial, insbesondere Tuben.

2. Verwendung nach Anspruch 1, **dadurch gekennzeichnet, dass** die Zubereitungen 0,001 bis - 10 Gew.-%, besonders bevorzugt 0,01 - 1 Gew.-%, an 4-n-Butylresorcin enthalten, bezogen auf die Gesamtzusammensetzung der Zubereitungen.

## Claims

1. Use of 4-n-Butylresorcinal for preventing or reducing the diffusion of one or more constituents of cosmetic emulsions into the container material, in particular tubes, surrounding the preparation.

2. Use according to Claim 1, **characterized in that** the preparations comprise 0.001 to 10% by weight, particularly preferably 0.01-1% by weight, of 4-n-Butylresorcinol, based on the total composition of the preparations.

## Revendications

1. Utilisation de 4-n-butylrésorcine pour empêcher ou réduire la diffusion d'un ou de plusieurs constituants d'émulsions cosmétiques dans le matériau du récipient entourant la préparation, en particulier des tubes.

2. Utilisation selon la revendication 1, **caractérisée en ce que** les préparations contiennent 0,001 à 10 % en poids, particulièrement préférablement 0,01 à 1 % en poids, de 4-n-butylrésorcine, par rapport à la composition totale des préparations.
